# EUROPEAN PATENT SPECIFICATION

(11) **EP 1 482 959 B1**
(45) Date of publication and mention of the grant of the patent: **02.04.2008**
(21) Application number: 03739405.3
(22) Date of filing: 17.02.2003
(51) Int. Cl.: A61K 35/74, A61P 17/00, A61P 17/04, A61P 37/08, A61P 17/10

(54) **COMPOSITIONS FOR TREATMENT OF SKIN DISORDERS**
ZUSAMMENSETZUNGEN ZUR BEHANDLUNG VON HAUTERKRANKUNGEN
COMPOSITIONS POUR LE TRAITEMENT DE TROUBLES CUTANES

(30) Priority: 15.02.2002 AU PS057102
(43) Date of publication of application: 08.12.2004
(73) Proprietor: Probiomics Limited, Eveleigh, NSW 1430 (AU)
(72) Inventor: CONWAY, Patricia, Lynne, La Perouse, NSW 2036 (AU); COLLIER, Paul, Anthony, Waterman, W.A. 6020 (AU)
(74) Representative: De Clercq, Ann G. Y.
(86) International application number: PCT/AU2003/000200
(87) International publication number: WO 2003/068250

(56) References cited:
- WO-A-00/71139
- WO-A-02/45726
- WO-A1-01/52912
- PATENT ABSTRACTS OF JAPAN vol. 2000, no. 07, 29 September 2000 (2000-09-29) & JP 2000 095698 A (NICHINICHI SEIYAKU KK), 4 April 2000 (2000-04-04)
- PATENT ABSTRACTS OF JAPAN & JP 09 263 539 A (NICHINICHI SEIYAKU KK) 07 October 1997
- PATENT ABSTRACTS OF JAPAN & JP 09 002 959 A (YAKULT HONSHA CO LTD) 07 January 1997
- ISOLAURI E. ET AL.: 'Probiotics in the management of atopic eczema' CLINICAL AND EXPERIMANTAL ALLERGY vol. 30, 2000, pages 1604 - 1610, XP001064231
- MAJAMAA H. ET AL.: 'Probiotics: a novel approach in the management of food allergy' THE JOURNAL OF ALLERGY AND CLINICAL IMMUNOLOGY vol. 99, no. 2, February 1997, pages 179 - 185, XP009022566
- KALLIOMAKI M. ET AL.: 'Probiotics in primary prevention of atopic disease; a randomised placebo-controlled trial' THE LANCET vol. 357, 07 April 2001, pages 1076 - 1079, XP004234216
- MURCH S.H.: 'Toll of allergy reduced by probiotics' THE LANCET vol. 357, 07 April 2001, pages 1057 - 1059, XP004234210
- MOMBELLI B. ET AL.: 'The use of probiotics in medical practice' INTERNATIONAL JOURNAL OF ANTIMICROBIAL AGENTS vol. 16, 2000, pages 531 - 536, XP001180028

## Description

### TECHNICAL FIELD

The invention relates to the field of skin disorder prevention and/or treatment, and in particular to probiotic bacteria and compositions containing same, which are useful in the prevention and/or treatment of skin disorders.

### BACKGROUND ART

Skin disorder such as dermatitis is an inflammatory condition of the skin characterised by erythema and pain or pruritis. Dermatitis is a heterogeneous group of skin disorders and various cutaneous lesions occur, each being potentially unique to a particular allergen, disease or infection. In many instances food or environmental factors may be the trigger however there are numerous skin conditions for which the cause or initial trigger are not known or which may be initiated by for example an allergen or an antigen but which persist despite preventing or discontinuing exposure to the allergen or antigen. Dermatitis may be chronic or acute and the treatment is usually specific to the cause, if known, or to the condition.

An example of a skin disorder of unknown cause is rosacea. This disorder may be defined as an inflammatory condition of the skin, presenting with a history of flushing and/or blushing along with clinical findings of erythema, edema, telangiectasia, papules, pustules, and nodules primarily of the face. Rosacea cannot be cured at present but popularly prescribed medical treatments for the condition include oral antibiotics such as tetracycline (1) and topical treatments such as 0.75% and 1.0% metronidazole gel (2, 3). Both these treatments have proved to maintain remission of moderate to severe rosacea. Other alternative treatments include isotretinion (accutane), a synthetic oral retinoid, which has been shown to produce results in patients who have been unresponsive to common forms of treatment for rosacea (4, 5).

Other skin conditions involving lesions with similar characteristics may in fact have a different cause and thus require different conventional treatment approaches. Whatever the cause of dermatitis, it is of great discomfort to the sufferer and treatment is often aggressive and may be associated with significant side effects.

Another example of a skin disorder which may be difficult to treat and which has a variety of causes is contact dermatitis, which may be triggered in sensitive subjects by skin contact with an external stimulus/agent. For example domestic animals frequently develop dermatitis of one type or another. Most commonly in dogs it is flea-induced dermatitis, which can progress to more severe skin infection if not treated rapidly. Hypersensitivity in dogs, especially to flea bite, is a major reason for the presentation of animals to community-based veterinary clinics. These animals are routinely treated with anti flea products, containing pesticides, and topical corticosteroids. Unfortunately these treatment options are less than ideal as fleas develop resistance to pesticides and prolonged use of corticosteroids carries with it the ever-present risk of Cushing's disease. A similar skin condition in humans may be triggered by house dust mite and represents a skin disorder arising from contact of skin with a substance or an agent capable of inducing an adverse reaction.

Clearly, alternative therapeutic options are required for skin disorders which may not have a known cause, are particularly difficult to treat or for which current therapies have significant limitations, or skin disorders arising from external contact with an antigen, an allergen or an agent capable of causing an adverse skin reaciton

There is therefore a need for improved treatments, whether prophylactic or therapeutic, for skin disorders such as those described above.

It is an object of the present invention to overcome or ameliorate at least one of the disadvantages of the prior art, or to provide a useful alternative.

### SUMMARY OF THE INVENTION

The present invention provides:
(i) use of a probiotic for the manufacture of a medicament for the prophylactic or therapeutic treatment of flea-induced dermatitis in a non-human mammal;
(ii) use as set forth in (i) above, wherein the treatment of flea-induced dermatitis results in treatment of one or more symptoms of said flea-induced dermatitis;
(iii) use as set forth in (ii) above, wherein said symptom is selected from pruritis, pain and erythema;
(iv) use as set forth in any one of (i) to (iii) above, wherein the flea-induced dermatitis has associated therewith visible lesions;
(v) use as set forth in any one of (i) to (iv) above, wherein the subject to be treated is a domestic pet or farm animal;
(vi) use as set forth in (v) above, wherein the subject to be treated is a cat or dog;
(vii) use as set forth in any one of (i) to (vi) above, wherein the probiotic is a lactic acid bacterium;
(viii) use as set forth in (vii) above, wherein the lactic acid bacterium is a Lactobacillus;
(ix) use as set forth in (viii) above, wherein the bacterium is Lactobacillus acidophilus or Lactobacillus fermentum;
(x) use as set forth in (ix) above, wherein the bacterium is Lactobacillus fermentum;
(xi) use as set forth in (x) above, wherein the Lactobacillus fermentum is Lactobacillus fermentum VRI-002;
(xii) use as set forth in any one of (i) to (xi) above, wherein the probiotic is administered as a pharmaceutical composition;
(xiii) use as set forth in any one of (i) to (xi) above, wherein the probiotic is administered as a food or feed preparation or as a food or feed supplement;
(xiv) use as set forth in any one of (i) to (xii) above, wherein the probiotic is administered topically;
(xv) use as set forth in any one of (i) to (xiii) above, wherein the probiotic is administered orally;
(xvi) use as set forth in any one of (i) to (xv) above, wherein the probiotic is administered with one or more pharmaceutically active agents used for treatment of skin disorders or dermatitis;
(xvii) use as set forth in (xvi) above, wherein the probiotic, or a composition containing said probiotic, is administered simultaneously or sequentially with one or more pharmaceutically active agents;
(xviii) use as set forth in any one of (i) to (xvii) above, wherein the amount of probiotic bacteria administered to a subject is in the range of about 10⁸ to about 10¹² bacteria;
(xix) use as set forth in (xviii) above, wherein the amount of probiotic bacteria administered to a human subject is about 10⁸ bacteria;
(xx) use as set forth in (xviii) above, wherein the amount of probiotic bacteria administered to a human subject is about 10¹⁰ bacteria;
(xxi) use as set forth in (xviii) above, wherein the amount of probiotic bacteria administered to a human subject is from about 10¹⁰ to about 10¹² bacteria;
(xxii) use as set forth in any one of (i) to (xxi) above, wherein the probiotic bacterium, or a composition containing said probiotic bacterium, is administered daily.

It has been unexpectedly found that certain probiotic bacteria, when administered to human subjects or animals in sufficient quantity, have the ability not only to down-regulate systemic and peripheral markers of inflammation and/or allergy but also to treat skin disorders which until now were difficult to treat or required therapeutic approaches which were associated with significant side-effects and variable efficacy. Further, the probiotic compositions of the present invention are also effective in treating skin disorders arising from contact of skin with a substance or an agent capable of causing a hypersensitivity or other adverse reaction.

According to one aspect the present specification describes a method for prophylactic or therapeutic treatment of a skin disorder by administering to a subject requiring such treatment a therapeutically or prophylactically effective amount of a probiotic.

According to another aspect the present specification describes a method of treatment of one or more symptoms of skin disorder by administering to a subject requiring such treatment an effective amount of a probiotic.

Preferably the skin disorder is dermatitis having associated therewith visible lesions. Even more preferably it is contact dermatitis, seborrheic dermatitis, actinic dermatitis, dermatitis caused by microbial infection and the like. A specific example of as particularly preferred skin disorder which can be treated by the herein described methods is rosacea. Another skin condition which can benefit from the herein described treatment is eczema. In mammals other than humans, for example domestic pets such as dogs, the preferred skin condition which can be treated by the methods and compositions of the present invention is flea-induced dermatitis. However, similar conditions in human subjects, for example house dust mite induced dermatitis or contact dermatitis, may also be treated by the methods and compositions described herein.

Preferably the symptoms of dermatitis which can be treated by compositions and methods of the present invention are selected from pruritis, pain and erythema.

Preferably, the probiotic is a lactic acid bacterium and even more preferred is *Lactobacillus fermentum* or *Lactobacillus acidophilus*. The most preferred strain of L. *fermentum* is the VRI-002 strain (obtained from University of New South Wales, Microbiology Culture Collection, Sydney, New South Wales, Australia; also deposited with Australian Government Analytical Laboratories, AGAL, of PO Box 385, Pymble NSW2073, Australia, on 12 December 2002 and given the accession number NM02/32959).

According to a further aspect the present specification describes a method of lowering levels of one or more immune markers of allergy and/or inflammation in a subject with dermatitis having elevated levels of said markers, by administering to a subject requiring such treatment an effective amount of a probiotic

Preferably the immune markers are selected from immunoglobulins and/or cytokines characteristic of allergic responses or of Th1 and Th2 T cell responses. For example suitable markers can be chosen from IgE, IL-4, IL-10, IL-12, interferon gamma, and the like.

Preferably, the probiotic is part of a composition, such as for example a food preparation, food supplement or a pharmaceutical preparation (eg, tablets, capsules, powders, liquid formulations and the like). The probiotic, or the composition containing said probiotic, may be administered by any known means but preferably it is administered orally. The probiotic, or the composition containing said probiotic, may also be advantageously administered topically, either by application to a mucosal surface, or by application directly to the affected skin areas.

It is also preferable that the probiotic, or the composition containing said probiotic, is administered at the onset of dermatitis or other skin disorder, or shortly thereafter. However, established skin lesions and conditions can be effectively treated by the methods of the present invention, as demonstrated in the examples.

The probiotic, or the composition containing said probiotic, may be administered in conjunction with one or more pharmaceutically active agents normally used for treatment of dermatitis. The probiotic, or the composition containing said probiotic, may be administered simultaneously (co-administered) with the other treatments or it may be administered sequentially in any order.

Preferably, the subject to be treated is selected from those subjects who are at high risk of exposure to allergens and/or antigens, or other agents, which may cause dermatitis, or individuals who have a family history or a genetic predisposition to developing dermatitis. Of course subjects already exhibiting overt signs of dermatitis, or having well advanced dermatitis can also advantageously be treated by the methods of the present invention.

The terms "subject" and "individual" are used interchangeably and in the context of the present invention include in their scope any mammal which can develop, or already has, dermatitis of whatever cause. Of course the preferred subjects for administration of the treatment of the present invention are humans, domestic pets and farm animals.

Preferably the amount of probiotic bacteria administered to a human subject is at least 10¹⁰ bacteria. More preferably the amount administered is from about 10¹⁰ to about 10¹² bacteria. The dosage may be administered daily as a single dose or may be divided into two or more doses to be taken at different intervals through the day. The treatment may be administered for short periods of time such as several days to several weeks, or it may be administered for prolonged periods of time such as months or years. It may also be administered in a life-long maintenance protocol. Intermitent dosage regime is also contemplated which may involve administration less frequent than daily, for example once, twice or three times per week, or similar dosage regime.

The required dosage amount will vary according to the severity of dermatitis, the cause of the condition, age of the subject and other standard clinical parameters which can be easily determined by routine procedures within the skill-set of those skilled in the art.

It is preferred that the probiotic or a composition containing the probiotc, be administered daily. Of course, it can be administered several times per day, or it may be administered Infrequently (for example every second or third day), depending on the progress of treatment of dermatitis, its cause and severity. These parameters can also be easily determined by those skilled in the art.

According to yet another aspect the present specification describes a use of a probiotic for the manufacture of a medicament for treating dermatitis or other skin disorders.

According to another aspect the present specification describes a dermatological composition comprising a therapeutically and/or prophylactically effective amount of probiotic, together with a pharmaceutically acceptable carrier, adjuvant, solvent or excipient.

Preferably the probiotic is a lactobacillus and more preferably it is *L. fermentum*. Even more preferred is *L. fermentum* VRI-002 strain.

The dosage form is preferably a tablet or a capsule, but it may also be a powder, liquid or paste/gel.

### BRIEF DESCRIPTION OF THE FIGURES

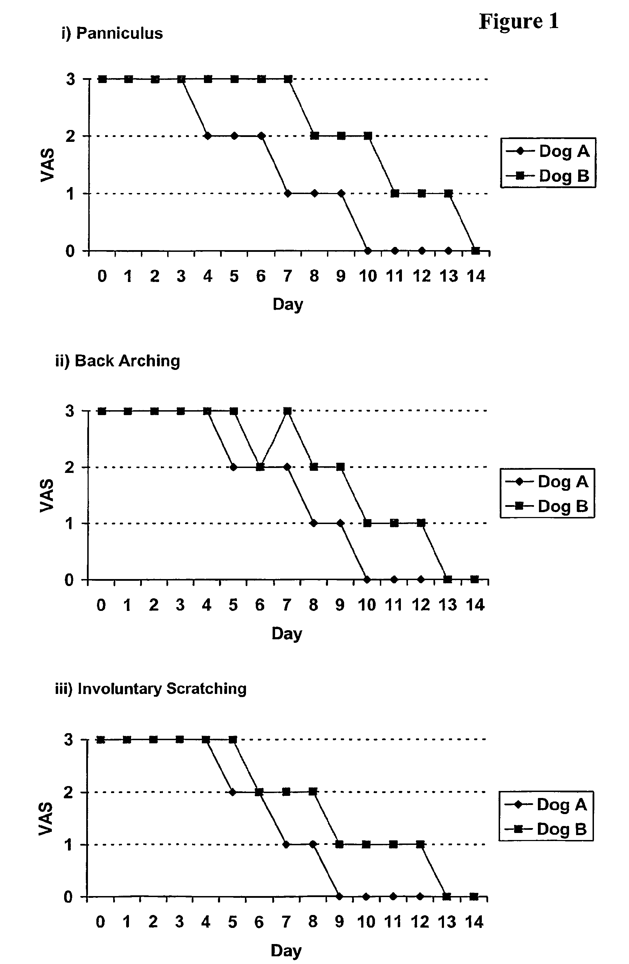

Figure 1. Mean VAS scores in dogs A and B for i) panniculus, ii) back arching and iii) involuntary scratching in response to oral administration of L. *fermentum* VRI-002. (Score: 0-Negative 1-low reactivity 2-moderate reactivity 3-high reactivity).

### DESCRIPTION OF THE PREFERRED EMBODIMENT

Whereas it may be expected that oral administration of probiotics could assist in food borne allergy treatment or treatment of GI tract infections, such an expectation is not realistic or appropriate when considering the treatment of skin conditions which are not associated with the oral intake of any particular infectious agent or allergen. The methods and compositions described herein have been developed for human and veterinary applications in the treatment of various forms of skin conditions, including dermatitis, and in particular skin conditions which have no known origin or trigger, or skin disorders which are difficult to treat or currently require treatments associated with significant side-effects. Whether used for treatment of humans or domestic animals, the underling principles are the same and advantageously the treatments described herein may be used irrespective of the cause of the skin condition or dermatitis.

Typically, the formulations containing probiotic bacteria, such as *Lactobacillus fermentum* VRI-002 strain, are administered orally. In the case of administration to a human subject, the dosage is in the form of capsules given twice daily. Typically the effective daily dosage is in the range of about 10⁸-10¹² bacteria and frequency of administration is once or twice daily. If treating animals such as dogs and cats, the appropriate dosage can be introduced in and/or with food.

The probiotic bacteria can be formulated into various compositions, both for veterinary and pharmaceutical use, typically capsules, tablets, powders, pastes and the like. Such formulations can be prepared by known means, using pharmaceutically acceptable carriers, excipients, solvents or adjuvants. Such procedures and ingredients are well know and amply described in standard texts and manuals, for example "Remington: The Science and Practice of Pharmacy", 1995, Mack Publishing Co. Easton, PA 18042, USA.

The probiotic bacteria may also be formulated into food or feed products and food or feed supplements by the usual well-known means. Such products are most suited for administration to animals such as domestic pets or farm animals.

The herein described methods and compositions are useful for
(1) treatment of skin disorders which have basis in, or are mediated by, a component of the immune system.
(2) treatment specifically of dermatitis of diverse aetiology
(3) treatment of dermatitis in all mammals, including humans, domestic pets and farm animals, particularly of dermatitis caused by skin contact (eg. contact dermatitis)
(4) prophylactic treatment of mammals in the prevention of occurrence or re-occurrence of dermatitis, particularly in subjects exposed to environments which may cause dermatitis or those who may be predisposed to developing dermatitis.

The specification further describes non-limiting examples. Examples 1 and 3 are particularly related to the invention as claimed.

### EXAMPLES

### Example 1: Preparation of Lactobacillus fermentum

Typically the L *fermentum* (VRI-002) was grown under industrial conditions in the following medium:

Yeast extract (20g), tryptone (0.5g), sodium acetate (1g), disodium hydrogen phosphate (Na2HPO4.2H2O; 0.2 g), potassium dihydrogen phosphate (0.7g), magnesium sulphate (MgSP4.7H2O; 0.7g), manganese sulphate (MnSO4.H2O; 0.06g), calcium chloride (0.05g), ferrous sulphate (FeSO4.7H2O; 0.005g), glucose (30g) and water (make up to one litre). After growth, cells were harvested by centrifugation and the slurry freeze dried using conventional cryoprotectants, according to known and well established methodology. The preparation of L. *fermentum* may be used immediately or stored for future use.

### Example 2: Case study 1 - treatment of rosacea in a human subject.

A male subject with a long history of rosacea, was treated with L. *fermentum* (VRI-002 strain). The subject was already receiving antibiotics at the time of initiation of treatment with L. *fermentum.*

The subject was treated twice daily, in the morning and at night, with 2 capsules on each occasion, each capsule included 7 x 10⁹ colony forming units (cfu's) of L. *fermentum.* The total daily dosage being 2.7 x 10¹⁰ cfu's of L. *fermentum .*

The subject ceased to take all medication apart from the L. *fermentum, 2* weeks prior to the doctor visit. At the visit, the rosacea had visibly improved in absence of antibiotics which were prescribed for the treatment of the condition. On cessation of L. *fermentum* treatment the condition returns but is suppressed with regular administration of L. *fermentum*.

### Example 3: Case study 2 - treatment of flea-induced dermatitis in dogs.

The aim of this study was to determine whether the oral administration of a particular probiotic strain of *Lactobacillus fermentum,* designated VRI-002, would reduce the clinical signs and symptoms of skin conditions induced by external contact of skin with an agent capable of or known to cause adverse skin reactions, such as contact dermatitis or the like. The model used for this purpose is flea-induced dermatitis in dogs.

Two household dogs (a Neopolitan Mastif and a Boxer-cross, mean weight 20-56 kg) living in the same household were chosen for the study. At the start of the experiment both animals exhibited numerous clinical signs of dermatitis, in particular intense pruritis, namely scratching, biting, alopecia at the base of the tail (one dog only). Both dogs were fed the same diet and lived together under identical conditions. The dogs were fed 10 mL of UHT skim milk using a 20 mL syringe containing *Lactobacillus fermentum* (prepared as described in example 1), at 5 x 10⁹ cfu daily (single dose) for 14 days with their evening meals. 10 mL of blood was collected before treatment and 3 days after treatment had ceased, for IgE and cytokine determination.

Clinical examinations by a qualified and practising veterinarian were performed each evening, immediately after the meal. The symptomatic parameters of pruritis measured were panniculus response, arching of back and involuntary lifting of hind leg and attempting to scratch the hair. An empiric visual analogue scale (VAS) was employed with the dog's reactions graded from 0 to 3 depending on the severity of reaction; 0 equating to a negative response through to 3 indicating a high reactivity.

### Immunological analysis

Flea antigen-specific IgE antibodies, as well as antibodies against numerous other antigens (see Table 1) were measured in serum using a commercial ELISA canine allergy test (Greer Laboratories, USA).

Semi-quantitative determination of IL-4, IL-5, IL-10 and IFN-γ mRNA gene expression in peripheral blood mononuclear cells was performed by RT-PCR, which is a well known and documented technique (Chamizo C et al. 2001; 83: 191-202). Histological assessment and cytokine analysis were determined on punch biopsies (2 mm deep skin sections) taken before and after treatment. Tissue sections were stained with hematoxylin and eosin (H&E) according to routine well know procedures, for the presence of inflammatory cells in the lesion. The levels of cytokine expression were determined by RT-PCR.

Figure 1 illustrates the individual clinical responses of the two dogs for each of the clinical symptoms of pruritis, across the 14 days of the study. As can be seen at the commencement of the study both animals exhibited overt signs of pruritis with both dogs exhibiting a VAS of 3 for all symptoms measured. Between days 4 and 8 each dog had started to show improvements in each of the clinical symptoms and by day 14 both dogs had ceased to exhibit clinical signs of the problem.

### Allergen reactivity IgE test

Serum samples were tested against a panel of common allergens, including fleas, for IgE antibody by ELISA. As shown in Table 1 below, there was a significant drop in allergen reactivity for IgE across the board after treatment with L. *fermentum* compared to baseline levels in both dogs. In particular, following treatment there was a reduction in IgE antibody for flea antigens, which correlated with a reduction in clinical symptoms common to dogs infected with fleas. Without wishing to be bound by theory, this may indicate a down-regulation of Th2 T-cell response associated with skin hypersensitivity.

**Table 1: IgE antibody against common allergens**

| | BEAUJOLAIS | | | JAEGER | | |
|---|---|---|---|---|---|---|
| ALLERGEN | %R Base | %R Test | | %R Base | %R Test | |
| couch grass | 77 | 63 | -18% | 110 | 100 | -9% |
| johnson grass | 80 | 66 | -18% | 104 | 90 | -13% |
| blue grass | 57 | 52 | -9% | 120 | 106 | -12% |
| ryegrass | 67 | 63 | -6% | 101 | 101 | 0% |
| cocksfoot | 74 | 62 | -16% | 103 | 90 | -13% |
| sweet vernel | 70 | 59 | -16% | 105 | 90 | -14% |
| yorkshire grass | 66 | 49 | -26% | 109 | 90 | -17% |
| canary grass | 65 | 61 | -6% | 99 | 100 | 1 % |
| paspalum species | 68 | 63 | -7% | 101 | 100 | -1% |
| prarie/broome grass | 63 | 59 | -6% | 94 | 111 | 18% |
| oat grass | 63 | 62 | -2% | 86 | 110 | 28% |
| english couch | 70 | 62 | -11% | 106 | 110 | 4% |
| dock/sorrell | 66 | 59 | -11% | 109 | 117 | 7% |
| pine | 67 | 56 | -16% | 99 | 91 | -8% |
| pigweed | 56 | 52 | -7% | 124 | 114 | -8% |
| chenopodium/fat hen | 59 | 56 | -5% | 113 | 117 | 4% |
| english plantain | 63 | 56 | -11% | 127 | 117 | -8% |
| short ragweed | 55 | 50 | -9% | 104 | 109 | 5% |
| western ragweed | 50 | 44 | -12% | 121 | 119 | -2% |
| dog fennel | 66 | 56 | -15% | 119 | 108 | -9% |
| housedust mite | 22 | 21 | -5% | 52 | 62 | 19% |
| housedust | 17 | 9 | -47% | 33 | 38 | 15% |
| fleas | 48 | 36 | -25% | 119 | 100 | -16% |
| wattle | 69 | 60 | -13% | 114 | 113 | -1% |
| casurina | 75 | 56 | -25% | 107 | 117 | 9% |
| elm | 58 | 44 | -24% | 119 | 119 | 0% |
| juniper | 63 | 43 | -32% | 80 | 112 | 40% |
| birch | 51 | 42 | -18% | 132 | 124 | -6% |
| olive | 62 | 49 | -21% | 116 | 108 | -7% |
| oak | 55 | 54 | -2% | 94 | 111 | 18% |
| melaleuca | 64 | 62 | -3% | 134 | 134 | 0% |
| privet | 71 | 52 | -27% | 113 | 130 | 15% |
| alternaria | 48 | 46 | -4% | 86 | 82 | -5% |
| aspegillus | 101 | 114 | 13% | 112 | 101 | -10% |
| cladospoium | 76 | 78 | 3% | 83 | 79 | -5% |

From the results of this study it would appear that oral administration of *Lactobacillus fermentum* in the dogs feed was effective in the treatment of this form of dermatitis and in particular in resolving the clinical symptoms of pruritis.

### Example 4: Case study 3 - treatment of acne.

Teenage (13 years old) identical male twins living in the same household, with developing acne were treated separately. Twin A was routinely administered with 2 capsules per day L *fermentum* VRI 002 in a capsule (10¹⁰ cfu per capsule) while twin B was not given any L *fermentum.* Both twins consumed the same diet and had similar routines. For the duration of the study (4 months), it was noted that Twin A had visibly less acne on the forehead and nose compared to twin B. The study was interrupted and twin B was given the L *fermentum* capsules as well because of the difference in the extent and severity of the acne.

### Example 5: Treatment of eczema.

### Case study 4:

### Patient: 2 ½ yr old girl:

- Suffering from eczema for the past 6 months
- Elbow and knee joints inflamed, buttocks inflamed and weepy
- Has taken 4 courses of antibiotics over 2 years
- Does not have regular bowel movements (every 3 days)
- Diet: very high in wheat (pasta, bread), dairy (2 bottles a day of cow's milk), tomatoes

Started oral adminisration of L. *fermentum* VRI-002, at a dosage rate of ½ capsule morning and night. Mother noticed improvement in the first week of treatment

Follow up visit 2 weeks later:
- Eczema almost gone from knee and elbow joint
- No longer on buttocks
- Did appear for the first time on upper thoracic spine
- Treatment: will continue with L. *fermentum* for 3 months

### Case study 5:

A 43 year old male patient presented with eczema in the form of an inflammed patch of skin on the palm of the left hand. Eczema was diagnosed approximately 13 years earlier. Treatment over several years included corticosteroid creams and the like.

Treatment with *L fermentum* VRI 002 commenced with 2 capsules per day of (10¹⁰ cfu per capsule) and continued for 3 months. No other treatment was administered. Following 1 week of treatment with *L fermentum* the lesion resolved almost completely. Beneficial effects on nails and GI tract were also reported.

### Case study 6:

Eighteen month old child (female) presented with eczema in the form of an inflammed area of skin of approximately 3 cm in diameter behind the right knee. The lesion has been in existence since shortly after birth and caused significant irritation and induced scratching. Treatment at presentation comprised application of a corticosteroid cream.

Treatment with the corticosteroid cream was suspended and treatment with *L fermentum* VRI 002 commenced with 1 capsule per day of (10¹⁰ cfu per capsule). No other treatment was administered. Following 1 week of treatment with *L fermentum* the lesion resolved completely. No side-effect of the L *fermentum* treatment were observed.

### Case study 7:

Patient: Adult female who has suffered from eczema since the age of 9 or so. It slowly got worse over the years. The patient also suffered from suspected irritable bowel and Candida. A number of creams have been tried for her eczema. Initially a weak steroid cream was used, then progressed to Advantan ointment, a strong steroid cream. The patient was going through 3-5 small tubes per month. When the eczema was really bad, the patient took steroid tablets and also tried anti-bacterial creams which made it worse. UV light treatment was attempted by a dermatologist. There was some initial success but eczema never resolved and the treatment increased the itch. The patient anti-histamines sometimes (2-3 different types at a time). After a month or so, the anti-histamine needed to be rotated as one at a time, they became ineffective. At one point a dermatologist diagnosed infected eczema and recommended that the patient be hospitalised. Intended treatment was with immunosuppressants but the patient refused. No further assistance or treatment was offered. The patient was started on *L. fermentum* treatment, 6 capsules in the morning and 6 at night (2-10 billion cfu of *L. fermentum* per capsule).

Following approximately one week of treatment there was a significant improvement in the severity of eczema. Although the treatment was not continued, there was a near total resolution of eczema in the first month following the treatment. Three months following the initial treatment the patient did not require further *L. fermentum* treatment.

Although the invention has been described with reference to specific examples, it will be appreciated by those skilled in the art that the invention may be embodied in many other forms.

### REFERENCES

1. Dahl MV, Katz HI, Krueger GG, Millikan LE, Odom RB, Parker F, Wolf JE Jr, Aly R, Bayles C, Reusser B, Weidner M, Coleman E, Patrignelli R, Tuley MR, Baker MO, Herndon JH Jr, Czernielewski JM. Topical metronidazole maintains remissions of rosacea. Archives of Dermatology 1998. 134: 679 - 83.
2. Dahl MV, Jarratt M, Kaplan D, Tuley MR, Baker MD. Once-daily topical metronidazole cream formulations in the treatment of the papules and pustules of rosacea. Journal of the American Academy of Dermatology 2001. 45: 723 - 30.
3. Feldman SR, Hollar CB, Gupta AK, Fleischer AB Jr. Women commonly seek care for rosacea: dermatologists frequently provide the care. Cutis 2001. 68: 156 - 60.
4. Mahrle G, Bauermeister-Jasso K, Enderer K. Roaccutan in acne and rosacea. Z Hautkr 1985. 60: 120, 125 - 34.
5. Hoting E, Paul E, Plewig G. The treatment of rosacea with isotretinoin. International Journal of Dermatology 1986. 25: 660 - 3.

## Claims

1. Use of a probiotic for the manufacture of a medicament for the prophylactic or therapeutic treatment of flea-induced dermatitis in a non-human mammal.

2. Use according to claim 1, wherein the treatment of flea-induced dermatitis results in treatment of one or more symptoms of said flea-induced dermatitis.

3. Use according to claim 2, wherein said symptom is selected from pruritis, pain and erythema.

4. Use according to any one of claims 1 to 3, wherein the flea-induced dermatitis has associated therewith visible lesions.

5. Use according to any one of claims 1 to 4, wherein the subject to be treated is a domestic pet or farm animal.

6. Use according to claim 5, wherein the subject to be treated is a cat or dog.

7. Use according to any one of claims 1 to 6, wherein the probiotic is a lactic acid bacterium.

8. Use according to claim 7, wherein the lactic acid bacterium is a Lactobacillus.

9. Use according to claim 8, wherein the bacterium is Lactobacillus acidophilus or Lactobacillus fermentum.

10. Use according to claim 9, wherein the bacterium is Lactobacillus fermentum.

11. Use according to claim 10, wherein the Lactobacillus fermentum is Lactobacillus fermentum VRI-002.

12. Use according to any one of claims 1 to 11, wherein the probiotic is to be administered as a pharmaceutical composition.

13. Use according to any one of claims 1 to 11, wherein the probiotic is to be administered as a food or feed preparation or as a food or feed supplement.

14. Use according to any one of claims 1 to 12, wherein the probiotic is to be administered topically.

15. Use according to any one of claims 1 to 13, wherein the probiotic is to be administered orally.

16. Use according to any one of claims 1 to 15, wherein the probiotic is to be administered with one or more pharmaceutically active agents used for treatment of skin disorders or dermatitis.

17. Use according to claim 16, wherein the probiotic, or a composition containing said probiotic, is to be administered simultaneously or sequentially with one or more pharmaceutically active agents.

18. Use according to any one of claims 1 to 17, wherein the amount of probiotic bacteria to be administered to a subject is in the range of about 10⁸ to about 10¹² bacteria.

19. Use according to claim 18, wherein the amount of probiotic bacteria to be administered to a human subject is about 10⁸ bacteria.

20. Use according to claim 18, wherein the amount of probiotic bacteria to be administered to a human subject is about 10¹⁰ bacteria.

21. Use according to claim 18, wherein the amount of probiotic bacteria to be administered to a human subject is from about 10¹⁰ to about 10¹² bacteria.

22. Use according to any one of claims 1 to 21, wherein the probiotic bacterium, or a composition containing said probiotic bacterium, is to be administered daily.

## Patentansprüche

1. Verwendung eines Probiotikums zur Herstellung eines Arzneimittels zur prophylaktischen oder therapeutischen Behandlung von durch Flohbefall hervorgerufener Hautentzündung bei einem Säugetier, außer beim Menschen.

2. Verwendung gemäß Anspruch 1, wobei die Behandlung der durch Flohbefall hervorgerufene Hautentzündung zu einer Behandlung eines oder mehrerer Symptome der durch Flohbefall hervorgerufenen Hautentzündung führt.

3. Verwendung gemäß Anspruch 2, wobei das Symptom aus Juckreiz, Schmerz und Hautrötungen gewählt ist.

4. Verwendung gemäß einem beliebigen der Ansprüche 1 bis 3, wobei die durch Flohbefall hervorgerufene Hautentzündung mit sichtbaren Läsionen verbunden ist.

5. Verwendung gemäß einem beliebigen der Ansprüche 1 bis 4, wobei es sich bei dem zu behandelnden Lebewesen um ein Haustier oder ein Nutztier handelt.

6. Verwendung gemäß Anspruch 5, wobei es sich bei dem zu behandelnden Lebewesen um eine Katze und einen Hund handelt.

7. Verwendung gemäß einem beliebigen der Ansprüche 1 bis 6, wobei es sich bei dem Probiotikum um ein Milchsäurebakterium handelt.

8. Verwendung gemäß Anspruch 7, wobei es sich bei dem Milchsäurebakterium um einen Lactobacillus handelt.

9. Verwendung gemäß Anspruch 8, wobei es sich bei dem Bakterium um Lactobacillus acidophilus oder Lactobacillus fermentum handelt.

10. Verwendung gemäß Anspruch 9, wobei es sich bei dem Bakterium um Lactobacillus fermentum handelt.

11. Verwendung gemäß Anspruch 10, wobei es sich bei dem Lactobacillus fermentum um Lactobacillus fermentum VRI-002 handelt.

12. Verwendung gemäß einem beliebigen der Ansprüche 1 bis 11, wobei das Probiotikum in Form einer pharmazeutischen Zusammensetzung zu verabreichen ist.

13. Verwendung gemäß einem beliebigen der Ansprüche 1 bis 11, wobei das Probiotikum in Form einer Nahrungs- oder Futtermittelzubereitung oder eines Nahrungs- oder Futtermittelzusatzes zu verabreichen ist.

14. Verwendung gemäß einem beliebigen der Ansprüche 1 bis 12, wobei das Probiotikum topisch zu verabreichen ist.

15. Verwendung gemäß einem beliebigen der Ansprüche 1 bis 13, wobei das Probiotikum oral zu verabreichen ist.

16. Verwendung gemäß einem beliebigen der Ansprüche 1 bis 15, wobei das Probiotikum mit einem oder mehreren pharmazeutischen Wirkstoffen, die zur Behandlung von störenden Hautveränderungen oder von Hautentzündung verwendet werden, zu verabreichen ist.

17. Verwendung gemäß Anspruch 16, wobei das Probiotikum, oder eine Zusammensetzung, welche das Probiotikum enthält, gleichzeitig oder aufeinander folgend mit einem oder mehreren pharmazeutischen Wirkstoffen zu verabreichen ist.

18. Verwendung gemäß einem beliebigen der Ansprüche 1 bis 17, wobei die Menge an probiotischen Bakterien, welche dem Lebewesen zu verabreichen ist, in der Größenordnung von ungefähr 10⁸ bis ungefähr 10¹² Bakterien liegt.

19. Verwendung gemäß Anspruch 18, wobei die Menge an probiotischen Bakterien, welche einem menschlichen Lebewesen zu verabreichen ist, ungefähr 10⁸ Bakterien beträgt.

20. Verwendung gemäß Anspruch 18, wobei die Menge an probiotischen Bakterien, welche einem menschlichen Lebewesen zu verabreichen ist, ungefähr 10¹⁰ Bakterien beträgt.

21. Verwendung gemäß Anspruch 18, wobei die Menge an probiotischen Bakterien, welche einem menschlichen Lebewesen zu verabreichen ist, ungefähr 10¹⁰ bis ungefähr 10¹² Bakterien beträgt.

22. Verwendung gemäß einem beliebigen der Ansprüche 1 bis 21, wobei das probiotische Bakterium, oder eine Zusammensetzung, welche das probiotische Bakterium enthält, täglich zu verabreichen ist.

## Revendications

1. Utilisation d'un probiotique pour la fabrication d'un médicament destiné au traitement prophylactique ou thérapeutique d'une dermatite induite par les puces chez un mammifère non humain.

2. Utilisation selon la revendication 1, dans laquelle le traitement de la dermatite induite par les puces résulte en le traitement d'un ou de plusieurs symptômes de ladite dermatite induite par les puces.

3. Utilisation selon la revendication 2, dans laquelle ledit symptôme est choisi parmi un prurit, la douleur et un érythème.

4. Utilisation selon l'une quelconque des revendications 1 à 3, dans laquelle des lésions visibles sont associées à la dermatite induite par les puces.

5. Utilisation selon l'une quelconque des revendications 1 à 4, dans laquelle le sujet à traiter est un animal domestique ou un animal de ferme.

6. Utilisation selon la revendication 5, dans laquelle le sujet à traiter est un chat ou un chien.

7. Utilisation selon l'une quelconque des revendications 1 à 6, dans laquelle le probiotique est une bactérie de l'acide lactique.

8. Utilisation selon la revendication 7, dans laquelle la bactérie de l'acide lactique est un lactobacille.

9. Utilisation selon la revendication 8, dans laquelle la bactérie est Lactobacillus acidophilus ou Lactobacillus fermentum

10. Utilisation selon la revendication 9, dans laquelle la bactérie est Lactobacillus fermentum.

11. Utilisation selon la revendication 10, dans laquelle Lactobacillus fermentum est Lactobacillus fermentum VRI-002.

12. Utilisation selon l'une quelconque des revendications 1 à 11, dans laquelle le probiotique doit être administré sous la forme d'une composition pharmaceutique.

13. Utilisation selon l'une quelconque des revendications 1 à 11, dans laquelle le probiotique doit être administré sous la forme d'une préparation alimentaire ou d'une préparation d'aliments pour animaux ou sous la forme d'un supplément alimentaire ou d'un supplément pour aliments pour animaux.

14. Utilisation selon l'une quelconque des revendications 1 à 12, dans laquelle le probiotique doit être administré par voie topique.

15. Utilisation selon l'une quelconque des revendications 1 à 13, dans laquelle le probiotique doit être administré par voie orale.

16. Utilisation selon l'une quelconque des revendications 1 à 15, dans laquelle le probiotique doit être administré avec un ou plusieurs agents pharmaceutiquement actifs utilisés pour le traitement des troubles cutanés ou d'une dermatite.

17. Utilisation selon la revendication 16, dans laquelle le probiotique, ou une composition contenant ledit probiotique, doit être administré(e) simultanément ou séquentiellement avec un ou plusieurs agents pharmaceutiquement actifs.

18. Utilisation selon l'une quelconque des revendications 1 à 17, dans laquelle la quantité de bactéries probiotiques à administrer à un sujet se trouve dans la plage d'environ 10⁸ à environ 10¹² bactéries.

19. Utilisation selon la revendication 18, dans laquelle la quantité de bactéries probiotiques à administrer à un sujet humain est d'environ 10⁸ bactéries.

20. Utilisation selon la revendication 18, dans laquelle la quantité de bactéries probiotiques à administrer à un sujet humain est d'environ 10¹⁰ bactéries.

21. Utilisation selon la revendication 18, dans laquelle la quantité de bactéries probiotiques à administrer à un sujet humain est d'environ 10¹⁰ à environ 10¹² bactéries.

22. Utilisation selon l'une quelconque des revendications 1 à 21, dans laquelle la bactérie probiotique, ou une composition contenant ladite bactérie probiotique, doit être administrée tous les jours.
